## Europäisches Patentamt
## European Patent Office
## Office européen des brevets

(11) Veröffentlichungsnummer: **0 311 961 A2**

(12) **EUROPÄISCHE PATENTANMELDUNG**

(21) Anmeldenummer: 88116805.8

(22) Anmeldetag: 11.10.88

(51) Int. Cl.⁴: **C07C 143/11**

(30) Priorität: 16.10.87 DE 3735056

(43) Veröffentlichungstag der Anmeldung:
19.04.89 Patentblatt 89/16

(84) Benannte Vertragsstaaten:
**BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Fikentscher, Rolf, Dr.**
**Von-Stephan-Strasse 27**
**D-6700 Ludwigshafen(DE)**
Erfinder: **Greif, Norbert, Dr.**
**Im Woogtal 3**
**D-6719 Bobenheim(DE)**
Erfinder: **Oppenlaender, Knut, Dr.**
**Otto-Dill-Strasse 23**
**D-6700 Ludwigshafen(DE)**
Erfinder: **Stork, Karl, Dr.**
**Reutersgarten 1**
**D-6840 Lampertheim(DE)**

(54) **Verfahren zur Herstellung von Ether- und Polyglykolethersulfonaten und nach diesem Verfahren hergestellte Produkte.**

(57) Die Erfindung betrifft ein Verfahren zur Herstellung von Ether- oder Polyglykolethersulfonaten durch Umsetzen von organischen alkoholischen oder phenolischen Verbindungen oder der entsprechenden Alkoxilate in wäßriger Lösung mit Vinylsulfonat in Gegenwart von 0,1 bis 3,0 Gew.% eines alkalischen Katalysators und gegebenenfalls zusätzlich eines Phasentransferkatalysators sowie nach diesem Verfahren hergestellte Ether- oder Polyglykolethersulfonate der allgemeinen Formel

$$R\text{-}O(C_nH_{2n}O)_z\text{-}CH_2CH_2SO_3X$$

worin
R = H, aliphatischer oder aromatischer Kohlenwasserstoffrest (evtl. mit Stickstoff als Heteroatom) mit 1 bis 30 C-Atomen
n = 2 bis 30
z = 0 bis 300 und
X = Alkalimetall

**Verfahren zur Herstellung von Ether- und Polyglykolethersulfonaten und nach diesem Verfahren hergestellte Produkte**

Die Erfindung betrifft Verfahren zur Herstellung von Ether- und Polyglykolethersulfonaten durch Umsetzen von organischen alkoholischen oder phenolischen Verbindungen oder der entsprechenden Alkoxilate mit organischen Sulfonatkomponenten sowie nach diesem Verfahren hergestellte Ether- und Polyglykolethersulfonate.

Bei derartigen Verfahrensprodeukten handelt es sich um anionische Tenside, d.h. grenzflächenaktive Netzmittel, die für viele Einsatzzwecke im technischen bzw. industriellen Bereich benötigt werden.

Die gängigen anionischen Tenside wie Alkylbenzolsulfonate, Olefinsulfonate und Paraffinsulfonate weisen häufig jedoch ungenügende Tensideigenschaften, vor allem aber eine schlechte Hartwasserbeständigkeit, auf.

Anionische Tenside mit einer Polyglykoletherkette zeigen hingegen stark verbesserte Eigenschaften gegenüber elektrolythaltigen wässrigen Medien, z.B. Alkylpolyglykolethersulfate, Natriumsalze von Schwefelsäurehalbestern von Polyglykolethern. Diese Produkte sind als Ester mit S-O-Bindungen jedoch bereits in schwach sauren Medien nicht hydrolysestabil. Gute Hartwasserbeständigkeit und Verseifungsstabilität sollten jedoch bei Polyglykolethersulfonaten gegeben sein, d.h. bei Produkten mit C-S-Bindungen.

$$R\text{-}O(C_nH_{2n}\,O)_x\text{-}(CH_2)_x\text{-}SO_3Na$$

Solche Produkte sind bekannt, jedoch sind die Synthesewege zu ihrer Herstellung verbesserungsbedürftig.

So lassen sich Polyglykolether (Alkoxilate) mit $SOCl_2$, $PCl_3$ oder $COCl_2$ über die entsprechenden Zwischenstufen in die Chloride der Polyglykolether überführen, die dann mit $Na_2SO_3$ zu den entsprechenden Sulfonaten umgesetzt werden. Diese Methodik ist jedoch präparativ sehr aufwendig, ferner entstehen korrosive Produkte ($SO_2$, $HCl$) oder aber größere Mengen an $NaCl$, die aus den Reaktionsmischungen entfernt bzw. abgetrennt werden müssen, zudem ist beim Arbeiten mit Phosgen besondere Vorsicht angebracht.

Andere Synthesewege haben ebenfalls Nachteile. So muß beim "Allyletherweg", zunächst durch Umsetzung der Alkoxilate mit Allylchlorid, der entsprechende Allylether hergestellt werden ($HCl$- bzw. $NaCl$-Anfall), an den in einer Folgereaktion $NaHSO_3$ addiert wird. Die Umsetzung von Polyglykolethern mit Propansulton zu den entsprechenden "Propylsulfonaten" ist aus toxikologischen und wirtschaftlichen Gründen wenig attraktiv.

Ein besserer Weg ist die aus der DE-A-27 48 721 bekannte Reaktion von Alkoxilaten mit Hydroxiethylsulfonat im alkalischen Medium. Hierbei muß jedoch zunächst das Hydroxiethylsulfonat aus Ethylenoxid und $NaHSO_3$ hergestellt werden. Die folgende Umsetzung mit OH-gruppenhaltigen Produkten ist jedoch nicht immer mit guten Ausbeuten möglich. Alkylpolyglykolether, Alkylphenole oder Alkanole reagieren unter diesen Bedingungen z.B. nicht oder mit niedrigen Ausbeuten.

Aufgabe der vorliegenden Erfindung war es, einen verbesserten Syntheseweg zur Schaffung von Ether- und Polyglykolethersulfonaten aufzufinden, der die oben angegebenen Nachteile nicht aufweist und von technisch leicht zugänglichen Produkten ausgeht.

Diese Aufgabe wurde durch ein Verfahren gemäß Patentansprüche 1 bis 3 gelöst.

Ether- oder Polyglykolethersulfonate sind an sich so bekannt, so daß sich weitere Erläuterungen erübrigen. Die zur Umsetzung gelangenden Ausgangsverbindungen, die organischen alkoholischen oder phenolischen Verbindungen, sind ebenfalls so bekannt, daß auf sie nicht besonders eingegangen werden muß. Das gleiche gilt für die Alkoxilate, die durch Umsetzen der Alkohole bzw. Phenole mit Epoxiden wie Ethylenoxid oder Propylenoxid erhalten werden.

Geeignete Ausgangsverbindungen sind:

$C_1$- bis $C_{30}$-, bevorzugt $C_4$- bis $C_{22}$-Alkanole (geradkettig oder verzweigt), Phenol, Kresole, Mono-, Di oder Tri-n- oder i-alkylphenole, z. B. Mono-, Di- oder Tri-tert-butylphenol, i-Octyl- oder Nonylphenol, Di-i-nonylphenol, n-oder i-Dodecylphenol, mit Alkylresten, die insgesamt 1 bis 100, insbesondere 4 bis 30 C-Atome enthalten können bzw. die entsprechenden Alkoxilate.

Diole wie Ethylenglykol, Propylenglykol, Bisphenol A oder Etherdiole oder Polyetherdiole wie Di-, Tri- oder Polyalkylenglykole, z.B. Produkte des Pluronictyps.

Entsprechende Triole (Glycerin, Trimethylolpropan) sowie andere Polyole (Pentaerythrit, Sorbit) und ihre Polyalkoxilate (Polyetherpolyole).

Für die alkalische oder sauer katalysierte Alkoxilierungsreaktion sind 1,2-Epoxide wie Ethylen-, Propylen- oder Butylenoxide (1,2-, iso- und 2,3-Butylenoxid) sowie längerkettige 1,2-Epoxide mit Alkylresten bis zu 30 C-Atomen geeignet, wie Styroloxid, Cyclohexenoxid etc..

Die Polyglykoletherketten können in Blockform

....ABABABAB....

oder aber statistisch verteilt (Mischbegasung mit Epoxidgemischen) vorliegen. AABABABABBBAAB etc..

Das Verfahren ist sowohl für (Alkyl-)Phenole, Alkanole als auch für ihre Polyglykoletherderivate anwendbar. Analoges gilt für die Alkoxilierungsprodukte anderer Produktklassen wie Amin- und Amidalkoxilate sowie alle Substanzklassen, die über eine funktionelle Gruppe mit aktiven H-Atomen verfügen, die eine Umsetzung mit Epoxiden zu den entsprechenden OH-Alkyl-Derivaten ermöglichen.

Besonders bevorzugt sind jedoch Polyglykolethersulfonate von Alkoholen, Phenolen, Alkylphenolen und ihren Alkoxilaten.

Bei der Umsetzung zu den entsprechenden Sulfonaten wird erfindungsgemäß mit Vinylsulfonat (Ethersulfonat) umgesetzt, das technisch leicht zugänglich ist und als wäßrige Lösung im Konzentrationsbereich von 30 bis 60 Gew.% erhältlich ist. Die Reaktion erfolgt in wäßriger Lösung, wobei die alkoholische Komponente in konzentrierter Form (100 %) eingesetzt wird. In vielen Fällen kann dabei der in der DE-A-27 48 721 beschriebene Temperaturbereich von ca. 180° C unterschritten werden. Bevorzugt wird jedoch im Temperaturbereich von 160 bis 180° C umgesetzt.

Bei der Umsetzung mit Vinylsulfonat liegen die Ausgangsprodukte in einem molaren Verhältnis von 1:1 bis 1:1,3 (bezogen auf reaktive alkoholische OH-Gruppen), bevorzugt in äquimolaren Mengen vor. Zur Erhöhung der Ausbeuten bzw. des anionischen bzw. nichtionischen Anteils im Reaktionsprodukt kann das Vinylsulfonat bzw. die alkoholische Komponente in bis zu 50 mol%igem Überschuß eingesetzt werden.

Es wird stets in Gegenwart von 0,1 bis 3, bevorzugt 0,5 bis 2,0 Gew.% eines alkalischen Katalysators gearbeitet, wobei sich die Gewichtsprozente auf die Alkoholkomponente beziehen. Gegebenenfalls wird in Gegenwart eines Phasentransferkatalysators, bevorzugt in Mengen von 0,2 bis 2,0 Gew.%, bezogen auf die Alkoholkomponente, gearbeitet. Bevorzugte Phasentransfer katalysatoren sind quarternäre Ammoniumsalze, insbesondere Methyltrioctylammoniumchlorid.

Der zusätzliche Einsatz eines Phasentransferkatalysators ist besonders bei Verwendung von verzweigten Alkanolen nützlich oder notwendig. Der alkalische Katalysator ist bevorzugt NaOH oder KOH.

Die erfindungsgemäß erhaltenen Produkte weisen die allgemeine Formel:

$$R-O(C_nH_{2n}\ O)_z-CH_2CH_2SO_3X$$

auf, wobei
R = H, aliphatischer oder aromatischer Kohlenwasserstoffrest (evtl. mit N als Heteroatomen) mit 1 bis 30, bevorzugt 1 bis 22 C-Atomen
n = 2 bis 30, bevorzugt 2 bis 4,
z = 0 bis 300, bevorzugt 0 bis 100
X = Alkalimetall, bevorzugt Na oder K

Die Produkte können unter anderem als Tenside und Hilfsmittel für den Wasch- und Reinigungsmittelsektor, die Färberei, die Erdölgewinnung, den Pflanzenschutzsektor etc. eingesetzt werden.

Allgemeine Herstellung:

Die jeweilige alkoholische Komponente wird mit äquimolaren Mengen Vinylsulfonat in Gegenwart von 0,1 bis 3 Gew.% Alkalihydroxid bei 180° C während 3 bis 5 Stunden umgesetzt.

Vor der Zugabe des Vinylsulfonats werden durch ein- bis zweistündiges Erhitzen im Vakuum (20 mm/Hg) auf 180° C die entsprechenden Alkalialkoholate der alkoholischen Komponenten hergestellt.

Beispiele: vgl. Tabelle

3

Tabelle

| Alkoholkomponente | O H Z | Vinylsulfonat | Mol-verhältnis | Reaktions-temperatur °C | Reaktions-zeit h | Alkylikatalysator (Gew.-% bez. auf Alkoholkomponente) | O H Z des Endpro-dukts |
|---|---|---|---|---|---|---|---|
| Glycerin | 1830 | VS | 1 : 1 | 140 | 2.5 | 1.0 (KOH) | 435 (505 Th.) |
| Glycerin | 1830 | VS | 1 : 2 | 140 | 2.5 | 1.1 (KOH) | 135 (159 Th.) |
| Glycerin | 1830 | VS | 1 : 3 | 140 | 2.5 | 1.3 (KOH) | 43 |
| i-Nonylphenol x 4.6 EO | 138 | VS | 1 : 1 | 180 | 6.0 | 1.2 (KOH) | 18 |
| i-Nonylphenol | 258 | VS | 1.1 : 1 | 180 | 3.0 | 0.8 (KOH) | 36 |
| i-Nonylphenol | 258 | VS | 1.5 : 1 | 180 | 5.5 | 1.7 (KOH) | 32 |
| Dodecylphenol | 220 | VS | 1.2 : 1 | 180 | 6.0 | 1.2 (KOH) | 30 |
| i-Decanol x 7 EO | 125 | VS | 1 : 1 | 180 | 8.5 | 1.7 (KOH) | 16 |
| i-Tridecanol | 276 | VS | 1.17 : 1 | 180 | 8.0 | 1.5 (KOH [1]) | 40 |
| i-Tridecanol x 4.1 EO | 150 | VS | 1 : 1.3 | 180 | 6.5 | 1.7 (KOH) | 12 |
| Bisphenol-A | 492 | VS | 1 : 2 | 150 | 10.0 | 1.8 (KOH) | 15 |
| Pluronic P 85 | 25 | VS | 1 : 2 | 180 | 4.5 | 1.6 (KOH) | 6 |

Anmerkung: Bei Alkoholen ohne Ethergruppierung kann der Reaktionsverlauf über die OH-Zahl und IR-Spektrum kontrolliert werden (Bandenintensität von C=C und -OH nach Reaktion abnehmend, zunehmende Intensität der Etherbande bei ca. 1150 cm$^{-1}$).

[1] 0.75 % KOH

0.75 % Phasentransferkatalysator (Methyltrioctylammoniumchlorid)

OHZ: OH-Zahl, bestimmt nach DIN 53240 und 53783 (Acetylierung und Verseifung)

EP 0 311 961 A2

## Ansprüche

1. Verfahren zur Herstellung von Ether- oder Polyglykolethersulfonaten durch Umsetzen von organischen alkoholischen oder phenolischen Verbindungen oder der entsprechenden Alkoxilate mit organischen Sulfonatkomponenten, dadurch gekennzeichnet, daß man in wäßriger Lösung mit Vinylsulfonat in Gegenwart von 0,1 bis 3,0 Gew.% eines alkalischen Katalysators und gegebenenfalls zusätzlich eines Phasentransferkatalysators umsetzt.

2. Verfahren nach Anspruch 1, dadurch gekennzeichnet, daß man in Gegenwart von 0,5 bis 2,0 Gew.% eines Katalysators umsetzt.

3. Verfahren nach Anspruch 1, dadurch gekennzeichnet, daß man in Gegenwart eines quarternären Ammoniumsalzes als Phasentransferkatalysator umsetzt.

4. Ether- oder Polyglykolethersulfonate nach Ansprüchen 1 bis 3 der allgemeinen Formel

$$R\text{-}O(C_nH_{2n}\ O)_z\text{-}CH_2CH_2SO_3X$$

worin

$R$ = H, aliphatischer oder aromatischer Kohlenwasserstoffrest (evtl. mit Stickstoff als Heteroatom) mit 1 bis 30 C-Atomen

$n$ = 2 bis 30

$z$ = 0 bis 300 und

$X$ = Alkalimetall

5. Ether- oder Polyglykolethersulfonate nach Anspruch 4 worin

$R$ = H, aliphatischer oder aromatischer Kohlenwasserstoffrest (evtl. mit Stickstoff als Heteroatom) mit 1 bis 22 C-Atomen

$n$ = 2 bis 4

$z$ = 0 bis 100 und

$X$ = Na oder K